# EUROPÄISCHE PATENTSCHRIFT

(11) **EP 2 853 217 B1**
(45) Veröffentlichungstag und Bekanntmachung des Hinweises auf die Patenterteilung: **31.08.2016**
(21) Anmeldenummer: 13186749.1
(22) Anmeldetag: 30.09.2013
(51) Int. Cl.: A61B 18/00, A61B 18/12

(54) **Chirurgisches Gerät mit verbessertem Netzmodul**
Surgical device with an improved power module
Appareil chirurgical doté d'un module d'alimentation amélioré

(43) Veröffentlichungstag der Anmeldung: 01.04.2015
(73) Patentinhaber: ERBE Elektromedizin GmbH, 72072 Tübingen (DE)
(72) Erfinder: Schulz, Florian, 72108 Rottenburg a.N. (DE)
(74) Vertreter: Rüger, Barthelt & Abel

(56) Entgegenhaltungen:
- EP-A1- 1 519 472
- EP-A2- 0 430 929
- WO-A1-2010/124785
- DE-A1-102004 010 769
- DE-T5-112009 001 250
- US-A1- 2012 083 779

## Beschreibung

Die Erfindung betrifft ein Chirurgiegerät, insbesondere ein HF-chirurgisches Gerät zur Speisung eines chirurgischen Instruments.

Elektrochirurgische Geräte weisen üblicherweise ein Netzteil auf, das das Gerät aus dem öffentlichen Netz mit elektrischer Leistung versorgt. Dazu offenbart die DE 11 2009 001250 T5 ein Gerät, dessen Netzteil einstellbare Wechselrichter zur Speisung von HF-Generatoren enthält. Der HF-Generator ist für die simultane Abgabe verschiedener HF-Ausgangsspannungswellenformen eingerichtet. Diese dienen alternativ zum Schneiden oder zum Koagulieren. Die Steuerung des Geräts erfolgt durch einen zentralen Baustein mit einem Benutzerinterface, wobei dieser zentrale Baustein sowohl das Netzteil als auch den HF-Generator steuert. Das Netzteil ist dabei anhand von im HF-Kreis auftretenden Ereignissen steuerbar, um seine Spannung zu erhöhen oder zu vermindern. Dazu sendet der zentrale Steuerbaustein eine entsprechende Gleichstromanforderung an das Netzteil.

Weiter ist aus der WO 2010/124785 A1 ein HF-Chirurgiegenerator sowie ein Verfahren zum Betrieb desselben bekannt, bei dem der Generator über einen Gleichspannungswandler aus einer Gleichspannung gespeist wird. Der Gleichspannungswandler ist eingangsseitig an einen Zwischenkreis angeschlossen, der mittels eines Kondensators C gepuffert ist. Zur Versorgung des Zwischenkreises dient ein Wechselspannungs/Gleichspannungs-Wandler. Eine zentrale Steuerschaltung steuert den Generator, nicht aber die Spannungswandler.

Aus der EP 1 519 472 A1 ist ein Gerät zur Speisung eines elektrochirurgischen Instruments bekannt, das die Merkmale des Oberbegriffs des Patentanspruchs offenbart. Eingangsseitig weist das Gerät einen Hochsetzsteller (PFC) auf, an den ein Leistungsversorgungsteil angeschlossen ist. Dieses speist einen Hochfrequenzgenerator, der von einer Steuerschaltung gesteuert wird. Die Steuerschaltung steuert auch das Leistungsversorgungsteil.

Davon ausgehend ist es Aufgabe der Erfindung, ein Konzept für ein elektrochirurgisches Gerät mit verbesserter Systemarchitektur zu schaffen.

Diese Aufgabe wird mit dem Elektrochirurgiegerät nach Anspruch 1 und ein Steuerverfahren nach Anspruch 15 gelöst:
Das erfindungsgemäße Elektrochirurgiegerät stellt eine vom Versorgungsnetz potentialgetrennte Betriebsspannung für eine Last, wie bspw. einen HF-Generator, mittels eines Hochsetzstellers und eines nachgeschalteten Leistungsversorgungsteils bereit. Eine Steuereinrichtung wirkt steuernd sowohl auf den Hochsetzsteller wie auch auf das Leistungsversorgungsteil ein. Der Hochsetzsteller bewirkt eine Leistungsfaktorkorrektur. Er dient dazu, einen möglichst nahezu sinusförmigen Strom aus dem Spannungsversorgungsnetz zu entnehmen und daraus eine Gleichspannung zu erzeugen (Zwischenkreisspannung). Die Zwischenkreisspannung wird von dem Leistungsversorgungsteil in eine Spannung zur Versorgung der Last, d.h. beispielsweise des HF-Generators, umgesetzt. Der Hochsetzsteller ist von einer PFC-Steuerschaltung gesteuert. Das Leistungsversorgungsteil ist von einer Betriebsteuerschaltung gesteuert. Die PFC-Steuerschaltung und die Betriebssteuerschaltung sind über eine digitale Kommunikationsschnittstelle miteinander verbunden. Die PFC-Steuerschaltung, das Leistungsversorgungsteil und die digitale Kommunikationsschnittstele bilden gemeinsam die Steuereinrichtung.

Vorrangige Aufgabe der Betriebssteuerschaltung ist die Steuerung der Last, d.h. beispielsweise des HF-Generators und des zugehörigen Leistungsversorgungsteils. Vorrangige Aufgabe der PFC-Steuerschaltung hingegen ist die Steuerung des Hochsetzstellers. Vorzugsweise kann die PFC-Steuerschaltung außerdem ein Kleinspannungsversorgungsteil steuern, das wie das Leistungsversorgungsteil an die Zwischenkreisspannung angeschlossen ist. Das Kleinspannungsversorgungsteil dient zur Erzeugung einer Kleinspannung von zum Beispiel 12V, die potentialmäßig von dem Spannungsversorgungsnetz getrennt ist.

Die digitale Kommunikationsschnittstelle zwischen der PFC-Steuerschaltung und der Betriebssteuerschaltung ermöglicht einen Datenaustausch zwischen beiden, wobei die PFC-Steuerschaltung und die Betriebssteuerschaltung ansonsten autonom arbeiten. Die PFC-Steuerschaltung enthält beispielsweise einen Mikrocontroller oder eine sonstige vorzugsweise programmierbar ausgebildete Steuereinrichtung. Sie kann programmierte Regeleigenschaften aufweisen. Dazu kann ein programmiertes Einschwingverhalten, eine einstellbare Strombegrenzung sowie die Unterspannungserkennung, die Überspannungserkennung, die Überstromerkennung und auch das Umschalten von Bauelementen je nach gemessener Eingangsspannung gehören. Bauelemente, die eingangsspannungsabhängig umgeschaltet werden können, sind insbesondere induktive Speicherelemente des Hochsetzstellers. Die Unterspannungserkennung, die Überspannungserkennung, die Überstromerkennung oder ähnliches kann zur Erzeugung von Signalen genutzt werden.

Die digitale Kommunikationsschnittstelle ermöglicht die Meldung solcher Ereignisse (Unterspannung, Überspannung, Überstrom und dergleichen) durch Übertragung von Signalen (Ereignissignalen) von der PFC-Steuerschaltung an die Betriebssteuerschaltung. Auf diese Weise kann die Betriebssteuerschaltung beispielsweise über einen Netzausfall informiert werden, bevor die Zwischenkreisspannung signifikant absinkt. In Reaktion darauf, kann sie alle größeren Energieverbraucher (Last, Display, Lüfter und dergleichen) abschalten und mit der verbleibenden, zum Beispiel auf Pufferkondensatoren des Zwischenkreises gespeicherter, Restenergie Datensicherung betreiben und aktive Controller in einen definierten Ruhezustand überführen.

Die PFC-Steuerschaltung und die Betriebssteuerschaltung sind vorzugsweise voneinander räumlich getrennt aufgebaut. Sie können dabei auf einer Platine oder auch in unterschiedlichen Bausteinen untergebracht sein. Die räumliche und funktionelle Trennung der PC-Steuerschaltung von der Betriebssteuerschaltung ermöglicht eine einfache galvanische Trennung und eine Spezialisierung der PFC-Steuerschaltung auf die Aufgaben bei der Steuerung des Hochsetzstellers und eventuell eines Kleinspannungsversorgungsteils während die Betriebssteuerschaltung auf die Steuerung der Last und des Leistungsversorgungsteils optimiert sein kann. Es können somit die PFC-Steuerschaltung und die Betriebssteuerschaltung als Module beispielsweise für verschiedene Anwendungs- oder Leistungsklassen bereitgehalten und jeweils durch die digitale Kommunikationsschnittstelle miteinander verbunden werden. Dies schafft einfache Adaptions- und Gestaltungsmöglichkeiten zum Aufbau verschiedener Leistungsversorgungsteile für verschiedene Aufgaben mittels typisierter Bausteine.

Die Last kann ein HF-Generator sein, der in verschiedenen Betriebsarten mit unterschiedlicher Leistungsaufnahme betreibbar ist. Zum Beispiel sind die Leistungsaufnahme der Last und die von ihr zu erzeugende HF-Spannung beim Schneiden typischerweise deutlich größer als beim Koagulieren. Die Betriebssteuerschaltung kann die Last, d.h. den HF-Generator steuern, um die verschiedenen Betriebsmodi zu veranlassen. Sie kann zugleich das Leistungsversorgungsteil an verschiedene Betriebsarten anpassen, indem es beispielsweise auf verschiedene gewünschte Spannungen eingestellt wird (z.B. 5 V ... 250 V, je nachdem, ob koaguliert oder geschnitten werden soll). Zugleich (oder zuvor) kann die Steuerschaltung über die digitale Kommunikationsschnittstelle ein Signal an die PFC-Steuerschaltung geben, um diese an unterschiedliche Leistungen anzupassen. Damit können Regelvorgänge, die ansonsten bei Lastsprüngen zur Konstanthaltung der Zwischenkreisspannung ablaufen, vermieden oder deutlich verkürzt werden. Die PFC-Steuerschaltung kann so vorausschauend gesteuert werden. Insbesondere ist es zweckmäßig, wenn die Betriebssteuerschaltung so ausgebildet ist, dass sie über die digitale Kommunikationsschnittstelle vor Umschalten der Betriebsarbeit ein Signal an die PFC-Steuerschaltung sendet, um diese auf die bevorstehende geänderte Leistungsaufnahme der Last einzustellen. Damit kann sichergestellt werden, dass in dem Zwischenkreis zu Beginn eines Schneidvorgangs die für den Anschnitt erforderliche Energie auch tatsächlich bereitgestellt ist und zur Verfügung steht.

Die PFC-Steuerschaltung kann wie erwähnt zusätzlich einen potentialtrennenden Gleichspannungs-GleichspannungsWandler steuern. Vorzugsweise kann dieser als potentialtrennender Sperrwandler ausgebildet sein. Weiter vorzugsweise weist dieser Sperrwandler einen Synchrongleichrichter auf. Die PFC-Steuerschaltung kann sowohl den primärseitigen elektronischen Schalter des Sperrwandlers als auch den Synchrongleichrichter steuern. Durch Hinterlegung entsprechender Kennlinien und Timing-Schemata in der PFC-Steuerschaltung kann erreicht werden, dass der Sperrwandler und der angeschlossene Synchrongleichrichter über einen weiten Lastbereich mit hohem Wirkungsgrad arbeiten und außerdem der elektronische primärseitige Schalter sicher in den Stromnulldurchgängen geschaltet wird. Entsprechendes gilt für den elektronischen Schalter des Synchrongleichrichters.

Bei dem Verfahren kann die Betriebssteuerschaltung vor einer Laständerung über die Kommunikationsschnittstelle ein Signal an die PFC-Steuerschaltung senden, um den Betrieb des Hochsetzstellers an die bevorstehende Änderung des Leistungsbedarfs der Last anzupassen. Dies verbessert das Betriebsverhalten des Leistungsversorgungsteils und eines angeschlossenen HF-Generators oder einer sonstigen Last. Auch können in dem Hochsetzsteller gewonnene Daten an die Betriebssteuerschaltung gemeldet werden, z.B. um ein geordnetes Abschalten des Chirurgiegeräts ohne Verlust von gemessenen Daten, Einstellungen und Parametern zu bewirken.

Die PFC-Steuerschaltung 29 kann bei stationärem Betrieb über den Ausgang des Kleinspannungsversorgungsteils mit Betriebsspannung versorgt werden. Dieses wird allerdings von der PFC-Steuerschaltung selbst gesteuert, so dass um den Anlauf zu beschleunigen oder überhaupt erst zu ermöglichen, eine Startschaltung vorgesehen sein kann. Vorzugsweise ist diese mit mindestens einem elektronischen Schalter versehen, der einen resistiven Strompfad von dem Gleichspannungszwischenkreis zu dem Betriebsspannungseingang der PFC-Steuerschaltung so lange freigibt, bis das Kleinspannungsversorgungsteil zuverlässig Betriebsspannung liefert. Sobald dies der Fall ist, wird die Startschaltung inaktiv. Somit werden ohmsche Verluste in ihrem zur temporären Versorgung dienenden Strompfad minimiert.

Weitere Einzelheiten vorteilhafter Ausführungsformen der Erfindung sind Gegenstand der Zeichnung, der Beschreibung oder von Unteransprüchen. Es zeigen:
Figur 1 ein Chirurgiegerät zur Speisung eines chirurgischen Instruments, in schematisierter Gesamtdarstellung.
Figur 2 das Chirurgiegerät nach Figur 1, in Blockdarstellung.
Figur 3 bis 6 Komponenten des Chirurgiegeräts nach Figur 1 und 2, in schematisierter Schaltplandarstellung.

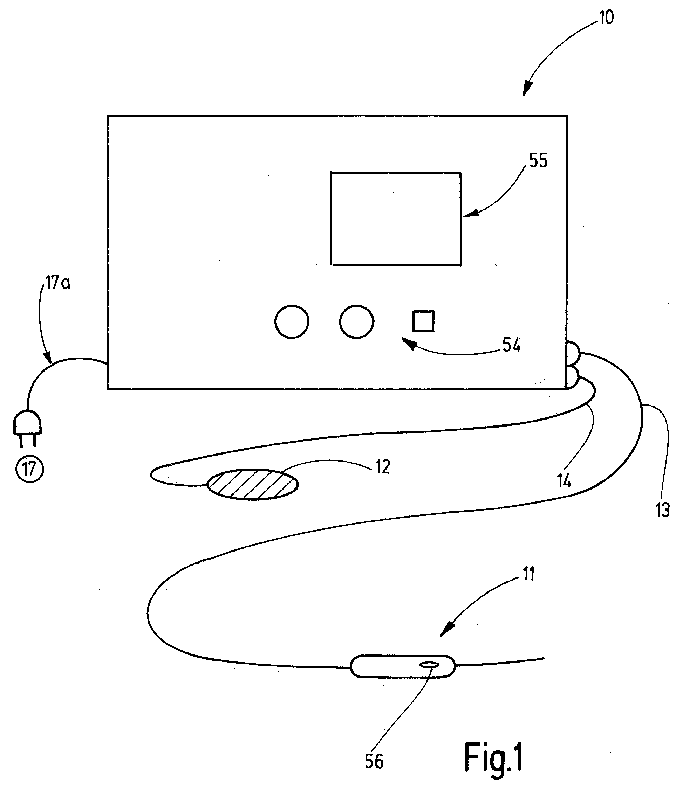

In Figur 1 ist ein Chirurgiegerät 10 veranschaulicht, das zur Speisung eines chirurgischen Instruments 11 dient. Das Instrument 11 kann ein offenchirurgische Instrument oder ein Instrument zur laparoskopischen Anwendung sein. Es kann, wie dargestellt, monopolar ausgebildet sein, wobei an dem zu behandelnden Patienten oder Objekt eine Neutralelektrode 12 zu befestigen ist. Das Instrument 11 kann auch bipolar ausgebildet sein. In diesem Fall entfällt die Neutralelektrode 12 und das Instrument 11 wird über eine zweiadrige Leitung versorgt. Das Instrument 11 und die Neutralelektrode 12 sind über Leitungen 13, 14 mit dem Chirurgiegerät 10 verbunden, um mit Strom und gegebenenfalls anderen Medien, wie bspw. Spülflüssigkeit oder dergleichen, versorgt zu werden.

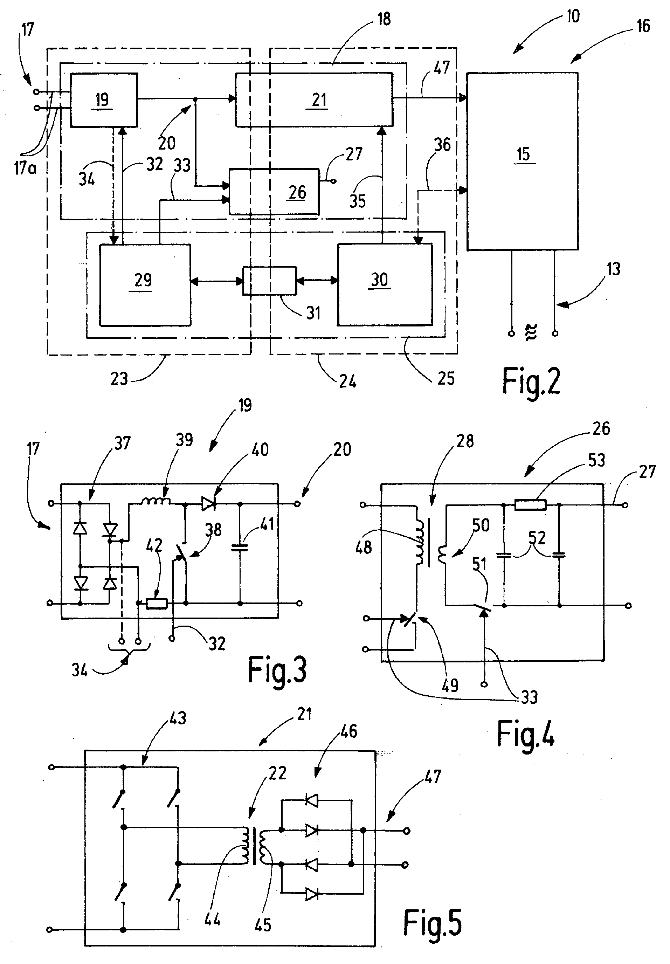

Das Chirurgiegerät 10 enthält zur Speisung des Instruments 11 beispielsweise einen HF-Generator 15, der aus dem Blockbild nach Figur 2 hervorgeht. Er bildet zusammen mit dem angeschlossenen Instrument 11 bzw. den im Stromkreis befindlichen Patienten eine elektrische Last 16. Die elektrische Leistung zum Betrieb dieser Last 16 entstammt einem Versorgungsnetz 17 an welches das Chirurgiegerät 10 über eine Netzleitung 17a angeschlossen ist. Zwischen der Last 16 (bzw. dem Generator 15) und dem über die Netzleitung 17a angeschlossenen Versorgungsnetz 17 ist ein Netzteil 18 angeordnet, dass die für die Last 16 erforderliche Betriebsspannung und den erforderlichen Betriebsstrom bereitstellt. Außerdem bewirkt das Netzteil 18 eine Potentialtrennung zwischen der Last 16 und dem Versorgungsnetz 17.

Aus Figur 2 sind wesentliche Baugruppen des Chirurgiegeräts 10 ersichtlich. Zu dem Netzteil 18 gehört ein Hochsetzsteller 19 zur Leistungsfaktorkorrektur. Der Hochsetzsteller 19 entnimmt über die Netzleitung 17a aus dem Versorgungsnetz 17 Strom und speist einen Gleichspannungszwischenkreis 20 mit einer Gleichspannung gewünschter Größe, die vorzugsweise größer als der Spitzenwert der zugeführten Netzspannung ist (bspw. 400 V). An den Gleichspannungszwischenkreis 20 ist ein Leitungsversorgungsteil 21 angeschlossen, das zur Speisung der Last 16 mit einer geeigneten, vorzugsweise innerhalb eines weiten Stellbereichs festlegbaren Spannung von z.B. 5 bis 250 Volt dient.

Das Leistungsversorgungsteil 21 ist schematisch aus Figur 5 ersichtlich. Es enthält einen Transformator 22, der der Potentialtrennung dient und somit das Netzteil 18 einem Netzspannungspotential führenden netzseitigen ersten Bereich 23 und einem vom Netzpotential getrennten patientenseitigen zweiten Bereich 24 zuordnet. Die beiden Bereiche 23, 24 umfassen sowohl das Netzteil 18 wie auch eine Steuereinrichtung 25.

Zu dem Netzteil 18 gehört zumindest optional ein Kleinspannungsversorgungsteil 26, das eingangsseitig mit dem Gleichspannungszwischenkreis 20 verbunden ist und an seinem Ausgang 27 eine geeignete Kleinspannung von zum Beispiel 12V bereitstellt. Das Kleinspannungsversorgungsteil 26 ist in Figur 4 gesondert veranschaulicht. Es enthält wiederum einen Transformator 28 zur Potentialtrennung, so dass das Kleinspannungsversorgungsteil 26 wie auch das Leistungsversorgungsteil 21 teilweise zu dem netzseitigen Bereich 23 und zu einem anderen Teil zu dem patientenseitigen Bereich 24 gehört.

Gleiches gilt für die Steuereinrichtung 25. Diese umfasst eine PFC-Steuerschaltung 29, die in dem netzseitigen Bereich 23 liegt. Außerdem umfasst die Steuereinrichtung 25 eine Betriebssteuerschaltung 30, die in dem patientenseitigen Bereich 24 liegt. Die PFC-Steuerschaltung 29 und die Betriebssteuerschaltung 30 sind über eine vorzugsweise bidirektionale Potential trennende digitale Kommunikationsschnittstelle 31 miteinander verbunden.

Die PFC-Steuerschaltung 29 steuert den Betrieb des Hochsetzstellers 19 und, sofern vorhanden, außerdem den Betrieb des Kleinspannungsversorgungsteils 26. Diese Wirkbeziehungen sind in Figur 2 durch Pfeile 32, 33 veranschaulicht. Außerdem kann von dem netzseitigen Bereich 23 des Netzteils 18 zum Beispiel von dem Hochsetzsteller 19 Information an die PFC-Steuerschaltung 29 geliefert werden, was Pfeil 34 andeutet.

Die Betriebssteuerschaltung 30 steuert zumindest das Leistungsversorgungsteil, das Pfeil 35 symbolisiert. Außerdem kann die Betriebssteuerschaltung 30 darauf eingerichtet sein, die Last 16 zu steuern sowie Informationen von der Last 16 zu erhalten, was Pfeil 36 andeutet. Zur Steuerung der Last 16 kann die Betriebssteuerschaltung 30 zum Beispiel die Betriebsarten vorgeben, wie bspw. Schneiden oder Koagulieren. Die Betriebssteuerschaltung 30 kann zum Beispiel Informationen über Spannungen und/oder Ströme eingangsseitig oder ausgangsseitig an dem HF-Generator 15 erhalten.

Figur 3 beschreibt eine Ausführungsform des Hochsetzstellers 19, unter Beschränkung auf seine Grundbestandteile. Eingangsseitig ist ein Netzgleichrichter 37 vorgesehen. An diesen schließt sich eine Reihenschaltung aus einem elektronischen Schalter 38 und einem induktiven Bauelement 39 an. Die Steuerelektrode des elektronischen Schalters 38, der beispielsweise als MOSFET ausgebildet ist, erhält Steuerimpulse über die durch den Pfeil 32 bezeichnete Leitung von der PFC-Steuerschaltung 29. Ausgangsseitig enthält der Hochsetzsteller eine Gleichrichterdiode 40 und einen Pufferkondensator 41. An geeigneter Stelle, beispielsweise am Ausgang des Netzgleichrichters 37, kann ein Netzspannungssignal abgegriffen und an die PFC-Steuerschaltung 29 geliefert werden. Außerdem kann an geeigneter Stelle ein Shunt 42 vorgesehen sein, dessen Spannungsabfall ebenfalls an die PFC-Steuerschaltung 29 geliefert wird (Pfeil bzw. Klammer 34 in Figur 3). An seinem Ausgang speist der Hochsetzsteller 19 den Gleichspannungszwischenkreis 20.

An den Gleichspannungszwischenkreis 20 sind das Leistungsversorgungsteil 21 nach Figur 5 sowie gegebenenfalls das Kleinspannungsversorgungsteil 26 nach Figur 4 angeschlossen.

Das Leistungsversorgungsteil 21 umfasst einen Wechselrichter 43, vorzugsweise ausgebildet als Vollbrückenwechselrichter. Dieser umfasst dann vier elektronisch steuerbare Schalter, die über die Wirkverbindung 35 von der Betriebssteuerschaltung 30 gesteuert sind. An den Wechselrichter 43 ist die Primärwicklung 44 des Transformators 22 angeschlossen. Seine Sekundärwicklung 45 ist mit einem Gleichrichterblock 46 verbunden, dessen Ausgang 47 die Last 16 mit einer Gleichspannung von zum Beispiel 5 bis 250 V versorgt. Der Gleichrichterblock 46 kann wie dargestellt durch eine Dioden-Brückenschaltung oder auch durch fremdgesteuerte Schalter als Synchrongleichrichter ausgebildet sein. Die Größe der Gleichspannung ist vorzugsweise durch entsprechende Steuerung des Wechselrichterblocks 43 durch die Betriebssteuerschaltung 30 steuerbar.

Das für kleinere Leistungen ausgelegte Kleinspannungsversorgungsteil 26 ist vorzugsweise als Sperrwandlerschaltung ausgebildet. Die Primärwicklung 48 des Transformators 28 ist mit einem elektronischen Schalter 49 in Reihe geschaltet. Die Steuerelektrode des elektronischen Schalters 49 ist über geeignete Impulsübertragungsmittel mit der PFC-Steuerschaltung 29 verbunden. Die Sekundärwicklung 50 des Transformators 28 ist über einen elektronischen Schalter 51 mit einem oder mehreren Pufferkondensatoren 52 verbunden, zwischen denen sich ein Siebwiderstand 53 oder auch eine entsprechende Drossel befinden kann. Die Steuerelektrode des elektronischen Schalters 51 ist über die durch den Fall 33 bezeichnete Wirkverbindung von der PFC-Steuerschaltung 29 gesteuert.

Die PFC-Steuerschaltung 29 kann durch einen Mikrocontroller gebildet sein, der das Timing des Ein- und Ausschaltens der elektronischen Schalter 49, 41 derart aufeinander abstimmt, dass der Schalter 51 einen Synchrongleichrichter bildet.

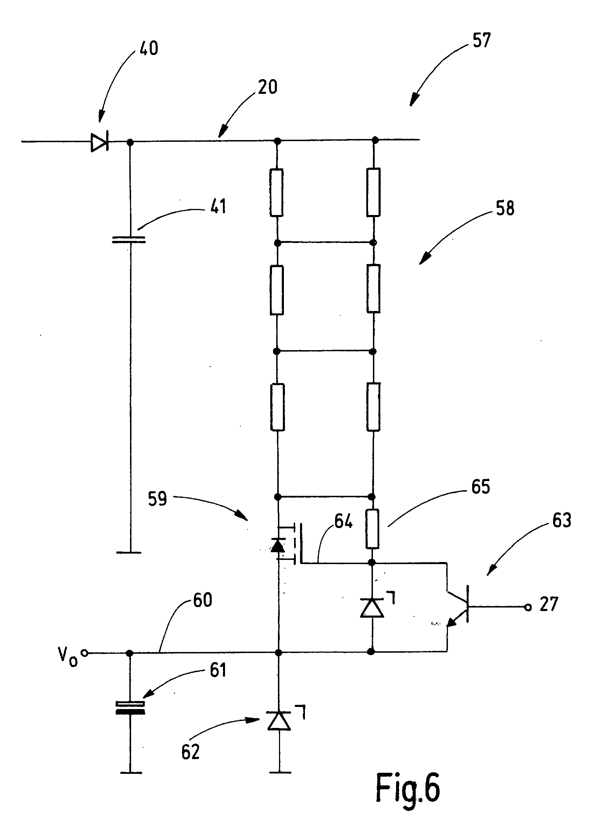

In Figur 6 ist eine Startschaltung 57 veranschaulicht, die dazu dient die PFC-Steuerschaltung 29 nach dem Start so lange mit Betriebsspannung zu versorgen bis das Kleinspannungsversorgungsteil 26 stabil arbeitet. Die Startschaltung 57 ist dabei an den Gleichspannungszwischenkreis 20 angeschlossen. Von dort erstreckt sich mindestens ein ohmscher Widerstand, vorzugsweise eine Widerstandskette 58, zu einem elektronischen Schalter 59. Die Reihenschaltung aus Widerstandskette 58 und elektronischem Schalter 59 bildet einen Strompfad von dem Gleichspannungszwischenkreis 20 zu einer kleinspannungsführenden Leitung 60, die mit dem Betriebsspannungseingang V₀ der PFC-Steuerschaltung 29 verbunden ist. Die Leitung 60 ist mittels eines Kondensators 61 gegen Masse gepuffert. Der Kondensator 61 ist mit einer Z-Diode 62 zur Spannungsbegrenzung überbrückt.

Die kleinspannungsführende Leitung 60 ist über einen Speisestrompfad mit dem Ausgang 27 des Kleinspannungsversorgungsteils 26 verbunden. Der Versorgungsstrompfad wird im vorliegenden Ausführungsbeispiel durch die Basis-Emitter-Diode eines oder mehrerer parallel geschalteter Transistoren, vorzugsweise npn-Transistoren 63 gebildet. Der Versorgungsstrompfad bildet den Steuereingang für den elektronischen Schalter 59. Sobald ein ausreichender Strom durch den Versorgungsstrompfad fließt, geht der elektronische Schalter 59 in seinen AUS-Zustand. Im Ausführungsbeispiel ist der elektronische Schalter 59 ein FeldeffektTransistor, dessen Gate 64 mit dem Kollektor des Transistors 63 sowie über einen Pull-Up-Widerstand 65 mit der Widerstandskette 58 verbunden ist. Außerdem kann sein Gate über eine spannungsbegrenzende Z-Diode mit seinem Source-Anschluss verbunden sein. Der Feldeffekttransistor leitet, wenn sein Gate 64 ausreichend positiv gegenüber seinem Source-Anschluss ist. Er sperrt, wenn das Gate 64 auf oder nahe bei Source-Potential liegt.

Weitere Eigenschaften der insoweit strukturell und von der Grundarchitektur hier beschriebenen Schaltung des Chirurgiegeräts 10 ergeben sich aus der nachfolgenden Funktionsbeschreibung:
Die Komponenten und Blöcke des Chirurgiegeräts sind so ausgebildet, dass die nachfolgend beschriebene Funktion erbracht wird oder erbracht werden kann:
   Es wird zunächst davon ausgegangen, dass das Chirurgiegerät 10 in Betrieb gesetzt worden ist.

Zunächst können wegen fehlender Steuerimpulse aus der PFC-Steuerschaltung 29 und der Betriebssteuerschaltung 30 weder beide Hochsetzsteller noch das Leistungsversorgungsteil 21, noch das Kleinspannungsversorgungsteil 26 arbeiten. Über die Diode 40 erhält der Pufferkondensator 41 gleichgerichtete Netzhalbwellen und lädt sich somit auf eine Spannung auf, die zunächst niedriger ist als die gewünschte Zwischenkreisspannung. Es aktiviert sich nun die Startschaltung 57, indem über die Widerstandskette 58 und dem Pull-Up-Widerstand 65 eine positive Spannung an das Gate 64 gelangt. Der Schalter 59 wird somit leitend (ON), wodurch ein durch die Widerstandskette 58 begrenzter Ladestrom auf den Kondensator 61 fließt und diesen lädt. Die sich aufbauende Spannung wird durch die Z-Diode 62 begrenzt. Somit wird der Anfangsbetrieb der PFC-Steuerschaltung 29 ermöglicht. Diese kann nun Steuerimpulse zu dem Hochsetzsteller 19 schicken, womit dieser seinen Betrieb aufnimmt und die gewünschte Zwischenkreisspannung auf dem Kondensator 41 aufbaut. Zugleich kann das Kleinspannungsversorgungsteil 26 unter der Kontrolle der PFC-Steuerschaltung 29 seinen Betrieb aufnehmen und an seinem Ausgang 27 die gewünschte und erforderliche Spannung bereitstellen. Es fließt ein Versorgungsstrom über die Basis-Emitter-Diode des Transistors 63 auf die Leitung 60, wodurch die Kollektor-Emitter-Strecke des Transistors 63 leitend wird. Das Gate 64 des Feldeffekttransistors wird dadurch auf Sourcepotential gezogen. Jedenfalls fällt die Gate-Source-Spannung unter die Schwellspannung des Feldeffekttransistors ab, wodurch dieser sperrt (OFF). Die Widerstandskette 58 wird dadurch strom- und verlustleistungslos. Der stationäre Betrieb der PFC-Steuerschaltung 29 ist damit erreicht. Die Widerstandskette kann so einerseits relativ niederohmig ausgelegt sein, um einerseits einen schnellen Start zu ermöglichen und den hohen Strombedarf der PFC-Steuerschaltung zu befriedigen, wobei andererseits durch die automatische Selbstabschaltung die im Betrieb auftretende Verlustleistung minimiert ist.

Nun setzt der Hochsetzsteller 19 die wellige gleichgerichtete Netzwechselspannung in eine Gleichspannung von zum Beispiel 400 V um. Der elektronische Schalter 38 wird dazu von der PFC-Steuerschaltung 29 mit einer deutlich über der Netzfrequenz liegenden Frequenz geöffnet und geschlossen. Es kann vorgesehen sein, dass die PFC-Steuerschaltung 29 die Spannung im Gleichspannungszwischenkreis 20 überwacht und das Tastverhältnis des Schalters 38 so reguliert, dass die gewünschte Gleichspannung eingehalten wird.

Es wird nun davon ausgegangen, dass die Betriebssteuerschaltung 30 einen bestimmten Betrieb des Instruments 11 vorzugeben hat, beispielsweise Koagulationsbetrieb. Diese Betriebsart kann beispielsweise durch ein oder mehrere Bedienelemente 54 am Gehäuse des Chirurgiegeräts 10 ausgewählt und durch ein oder mehrere Anzeigeeinrichtungen 55 signalisiert werden. Außerdem kann die Anzeigeeinrichtung 55 dazu ausgelegt sein, weitere Parameter wie Strom, Spannung, Koagulationsdauer oder dergleichen anzuzeigen.

Setzt der Anwender nun das Instrument am Anwendungsort ein und betätigt er beispielsweise ein Bedienelement 56 am Handgriff des Instruments 11, ist zum Beispiel der Generator 15 zu aktivieren. Die Betriebssteuerschaltung 30 stellt zuvor über die Wirkverbindung 35 das Leistungsversorgungsteil 21 so ein, dass an dem Ausgang 47 die zur Koagulation erforderliche Spannung ansteht. Dies erfolgt durch entsprechende Taktung der Schalter des Wechselrichters 43. Über die Wirkverbindung 36 kann dann der Generator 15 aktiviert und seine Parameter beispielsweise die Größe des Stroms und/oder die Größe der abgegebenen Spannung überwacht werden. Der HF-Generator 15, die Betriebssteuerschaltung 30 und das Leistungsversorgungsteil 21 können so eine Regelschleife bilden, um die gewünschten Parameter kontrolliert einzuhalten oder nach vorgebbaren Funktionen zu steuern.

Die Betriebsteuerschaltung 30 kann auch andere Betriebsarten steuern, beispielsweise Schneidbetrieb mit Anschnitt im feuchten Milieu. Hierzu ist erhöhte Leistung erforderlich. Während die Betriebssteuerschaltung 30 die Regulierung der Spannung an dem Ausgang 47 über Beeinflussung des Leistungsversorgungsteils 21 bewirkt, kann sie die bevorstehende erhöhte Leistungsanforderung über die Kommunikationsschnittstelle 31 an die PFC-Steuerschaltung 29 melden. Diese kann darauf eingerichtet sein, den über den Shunt 42 gemessenen Eingangsstrom kurzzeitig zu erhöhen. Die PFC-Steuerschaltung weicht dazu von ihrem sonst eingenommenen Betrieb der Konstanthaltung der Spannung im Gleichspannungszwischenkreis 20 ab. Diese wird normalerweise konstant ausgeregelt, indem die Spannung gemessen und durch die PFC-Steuerschaltung 29 mit einem Sollwert verglichen wird. Aus der Spannungsdifferenz wird ein Stromsollwert errechnet, der mit dem an dem Shunt 42 gemessenen tatsächlichen Strom verglichen wird. Bei stationärem Betrieb wird der Schalter 38 so angesteuert, dass der tatsächliche Strom dem Sollstrom entspricht.

In Vorwegnahme und Vorbereitung eines transienten Vorgangs, insbesondere einer sprungartigen Zunahme des Leistungsbedarfs der Last 16, kann die PFC-Steuerschaltung 29 zu dem Stromsollwert einen Offset hinzuaddieren und für eine gegebene oder gewählte Zeit einhalten. Dadurch fördert der Hochsetzsteller 19 mehr Energie in den Gleichspannungszwischenkreis 20, die nun für den Leistungsversorgungsteil 21 und der Last 16 zur Verfügung steht, beispielsweise um einen Anschnittvorgang zu bewirken.

In dieser Konfiguration ist es möglich, vorausschauend zu bevorstehenden Laständerungen zu agieren. Dadurch werden Spannungseinbrüche vermieden, die im Gleichspannungszwischenkreis 20 sonst auftreten könnten und durch die Spannungsregelung ausgeglichen werden müssten, wenn auf Lastsprünge lediglich reagiert würde.

Die Kommunikationsschnittstelle 31 zwischen der PFC-Steuerschaltung 29 und der Betriebssteuerschaltung 30 ermöglicht darüber hinaus weitere vorteilhafte Verhaltensweisen des Chirurgiegeräts 10. Beispielsweise kann bei einem Netzausfall, d.h. Wegfall der Netzspannung an der Netzleitung 17, dies über die durch den Pfeil 34 bezeichnete Wirkverbindung sofort der PFC-Steuerschaltung 29 und von dieser über die Kommunikationsschnittstelle 31 der Betriebssteuerschaltung 30 mitgeteilt werden. Eine mögliche Reaktion ist in einem solchen Fall, dass die Betriebssteuerschaltung 30 den Leistungsversorgungsteil 21 sofort stillsetzt, um einen möglichst langen Weiterbetrieb des Kleinspannungsversorgungsteils 26 aus der in Gleichspannungszwischenkreis 20 zum Beispiel auf dem Pufferkondensator 41 gespeicherten Energie zu ermöglichen. Der wenigstens Teile einer Sekunde oder auch mehrere Sekunden mögliche Weiterbetrieb des Kleinspannungsversorgungsteils 26 ermöglicht es dann, auf nicht weiter veranschaulichten Komponenten, wie insbesondere Speichern und Rechenbausteinen vorhandene Daten und Einstellwerte abzuspeichern und den Betrieb dann geordnet zu beenden. Energiezehrende Komponenten, wie insbesondere die Anzeigeeinrichtung 55, die Last 16 oder dergleichen, werden hingegen sofort stillgesetzt.

Das erfindungsgemäße Chirurgiegerät 10 weist ein Netzteil 18 auf, das mindestens einen Hochsetzsteller 19 und mindestens einen Leistungsversorgungsteil 21 enthält. Der Hochsetzsteller 19 oder bevorzugterweise das Leistungsversorgungsteil 21 bewirkt eine Potentialtrennung. Zwischen beiden ist ein Gleichspannungszwischenkreis 20 vorgesehen. Der Hochsetzsteller 19 wird von einer eigenen PFC-Steuerschaltung 29 betrieben. Das Leistungsversorgungsteil 21 wird hingegen von der Betriebsteuerschaltung 30 betrieben, die den allgemeinen Betrieb des Chirurgiegeräts 10 steuert. Zwischen der PFC-Steuerschaltung 29 und der Betriebssteuerschaltung 30 ist eine digitale Kommunikationsschnittstelle 31 vorgesehen, über die die Betriebssteuerschaltung 30 zusätzliche Informationen aus dem Hochsetzsteller 19 erhalten oder an diesen übergeben kann. Damit ist eine besonders schnelle und sichere Reaktion bei Netzspannungsausfall möglich. Außerdem kann der Hochsetzsteller vorausschauend unter Vorwegnahme bevorstehender Laständerungen betrieben werden. Dadurch können Betriebseigenschaften des Chirurgiegeräts 10 verbessert werden, beispielsweise hinsichtlich des Anschnitts oder dergleichen.

### Bezugszeichenliste:

- 10: Chirurgiegerät
- 11: Instrument
- 12: Neutralelektrode
- 13: Leitung zum Instrument 11
- 14: Leitung zur Neutralelektrode 12
- 15: HF-Generator
- 16: Last
- 17: Versorgungsnetz
- 17a: Netzleitung
- 18: Netzteil
- 19: Hochsetzsteller PFC
- 20: Gleichspannungszwischenkreis
- 21: Leistungsversorgungsteil
- 22: Transformator
- 23: netzseitiger Bereich
- 24: patientenseitiger Bereich
- 25: Steuereinrichtung
- 26: Kleinspannungsversorgungsteil
- 27: Ausgang des Kleinspannungsversorgungsteils 26
- 28: Transformator des Kleinspannungsversorgungsteils 26
- 29: PFC-Steuerschaltung
- 30: Betriebssteuerschaltung
- 31: Kommunikationsschnittstelle
- 32 - 36: Pfeile
- 37: Netzgleichrichter
- 38: Schalter
- 39: induktives Bauelement
- 40: Diode
- 41: Pufferkondensator
- 42: Shunt
- 43: Wechselrichter
- 44: Primärwicklung des Transformators 22
- 45: Sekundärwicklung des Transformators 22
- 46: Gleichrichterblock
- 47: Ausgang des Gleichrichterblocks 46
- 48: Primärwicklung des Transformators 28
- 49: elektronsicher Schalter
- 50: Sekundärwicklung des Transformators 28
- 51: elektronischer Schalter
- 52: Pufferkondensatoren
- 53: Siebwiderstand
- 54: Bedienelemente
- 55: Anzeigeeinrichtungen
- 56: Bedienelement
- 57: Startschaltung
- 58: Widerstandskette
- 59: elektronischer Schalter
- 60: kleinspannungsführende Leitung
- 61: Kondensator
- 62: Z-Diode
- 63: Transistor
- 64: Gate
- 65: Pull-Up-Widerstand

## Patentansprüche

1. Chirurgiegerät (10) mit einer Last (16), insbesondere HF-chirurgisches Gerät, zur Speisung eines chirurgischen Instruments (11),
mit einem Hochsetzsteller (19), der dazu eingerichtet ist, eine Netzwechselspannung in eine Zwischenkreisspannung umzusetzen und einen Gleichspannungszwischenkreis (20) zu speisen,
mit einem Leistungsversorgungsteil (21), das einen an den Zwischenkreis (20) angeschlossenen Wechselrichter (43) und einen Transformator (22) aufweist, der eine an den Wechselrichter (43) angeschlossene Primärwicklung (44) und eine Sekundärwicklung (45) zur Speisung der Last (16) aufweist,
mit einer Steuereinrichtung (25), die eine steuernd an das Leistungsversorgungsteil (21) angeschlossene Betriebssteuerschaltung (30) aufweist,
**dadurch gekennzeichnet, dass**
die Steuereinrichtung (25) außerdem eine steuernd an den Hochsetzsteller (19) angeschlossene PFC-Steuerschaltung (29) aufweist und
dass die PFC-Steuerschaltung (29) und die Betriebssteuerschaltung (30) über eine digitale Kommunikationsschnittstelle (31) miteinander verbunden sind.

2. Chirurgiegerät (10) nach Anspruch 1 mit der Last (16), **dadurch gekennzeichnet, dass** die Betriebssteuerschaltung (30) außerdem steuernd an die Last (16) angeschlossen ist.

3. Chirurgiegerät (10) nach Anspruch 2, **dadurch gekennzeichnet, dass** die Last (16) in verschiedenen Betriebsarten betreibbar ist, in denen sie unterschiedliche Leistungsaufnahmen aufweist.

4. Chirurgiegerät (10) nach Anspruch 2, **dadurch gekennzeichnet, dass** die Betriebssteuerschaltung (30) darauf eingerichtet ist, die Betriebsart der Last (16) vorzugeben und dass die Betriebssteuerschaltung (30) weiter darauf eingerichtet ist, die Leistungsversorgungseinheit (21) und die PFC-Steuerschaltung (29) der Betriebsart der Last (16) entsprechend zu steuern.

5. Chirurgiegerät (10) nach Anspruch 4, **dadurch gekennzeichnet, dass** die Betriebssteuerschaltung (30) darauf eingerichtet ist, die Leistungsversorgungseinheit (21) vor Umschalten der Betriebsart auf die bevorstehende geänderte Leitungsaufnahme der Last (16) einzustellen.

6. Chirurgiegerät (10) nach einem der vorstehenden Ansprüche, **dadurch gekennzeichnet, dass** die digitale Kommunikationsschnittstelle (31) potentialtrennend ausgebildet ist.

7. Chirurgiegerät (10) nach einem der vorstehenden Ansprüche, **dadurch gekennzeichnet, dass** die digitale Kommunikationsschnittstelle (31) bidirektional arbeitend ausgebildet ist.

8. Chirurgiegerät (10) nach Anspruch 4 oder 5, **dadurch gekennzeichnet, dass** die Betriebssteuerschaltung (30) darauf eingerichtet ist, die PFC-Steuerschaltung (29) über die digitale Kommunikationsschnittstelle (31) vor Umschalten der Betriebsart auf die bevorstehende geänderte Leitungsaufnahme der Last (16) einzustellen.

9. Chirurgiegerät (10) nach einem der vorstehenden Ansprüche, **dadurch gekennzeichnet, dass** an den Zwischenkreis (20) ein Kleinspannungsversorgungsteil (26) angeschlossen ist, das ein potentialtrennender Gleichspannungs-Gleichspannungs-Wandler ist.

10. Chirurgiegerät (10) nach Anspruch 9, **dadurch gekennzeichnet, dass** der potentialtrennende Gleichspannungs-Gleichspannungs-Wandler einen Fluss- oder Sperrwandler mit einem Synchrongleichrichter (51) aufweist.

11. Chirurgiegerät (10) nach Anspruch 9, **dadurch gekennzeichnet, dass** das Kleinspannungsversorgungsteil (26) von der PFC-Steuerschaltung (29) gesteuert ist.

12. Chirurgiegerät (10) nach Anspruch 10, **dadurch gekennzeichnet, dass** der Fluss- oder Sperrwandler mindestens einen elektronischen Schalter (49) aufweist, der mit einer Primärwicklung (48) eines potentialtrennenden Transformators (28) in Reihe geschaltet ist, wobei der Schalter (49) von der PFC-Steuerschaltung (29) getaktet ist.

13. Chirurgiegerät (10) nach Anspruch 10, **dadurch gekennzeichnet, dass** der Synchrongleichrichter einen elektronischen Schalter (51) mit einer Steuerelektrode aufweist, die mit Schaltimpulsen beaufschlagt ist, die von der PFC-Steuerschaltung (29) erzeugt sind.

14. Chirurgiegerät (10) nach einem der vorstehenden Ansprüche, **dadurch gekennzeichnet, dass** die PFC-Steuerschaltung (29) über eine selbstabschaltende Startschaltung (57) mit dem Gleichspannungszwischenkreis (20) verbunden ist.

15. Verfahren zur Bereitstellung von Betriebsleistung für eine Last (16), insbesondere eines HF-Generators (15) zur Speisung eines chirurgischen Instruments (11),
bei dem mit einem Hochsetzsteller (19), der eine Netzwechselspannung in eine Zwischenkreisspannung umsetzt, ein Zwischenkreis (20) gespeist wird, und
bei dem mit einem Leistungsversorgungsteil (21), dem aus dem Zwischenkreis (20) elektrische Energie zugeführt wird, die Last (16) gespeist wird,
**dadurch gekennzeichnet, dass** eine steuernd an den Hochsetzsteller (19) angeschlossene PFC-Steuerschaltung (29) über eine digitale Kommunikationsschnittstelle (31) mit Steuersignalen versorgt wird, die aus einer Betriebssteuerschaltung (30) stammen und Laständerungen kennzeichnen, und Statussignale an die Betriebssteuerschaltung (30) sendet.

## Claims

1. Surgical device (10) having a load (16), in particular an HF surgical device, for supplying a surgical instrument (11),
having a boost converter (19) which is configured to convert mains alternating voltage into an intermediate circuit voltage and to supply a DC voltage intermediate circuit (20),
having a power supply part (21) which has an inverter (43) connected to the intermediate circuit (20) and a transformer (22) which has a primary winding (44) connected to the inverter (43) and a secondary winding (45) for supplying the load (16),
having a control device (25) which has an operation control circuit (30) connected to the power supply part (21) in a controlling manner,
**characterised in that**
the control device (25) furthermore has a PFC control circuit (29) connected to the boost converter (19) in a controlling manner and
the PFC control circuit (29) and the operation control circuit (30) are connected to each other via a digital communications interface (31).

2. Surgical device (10) according to claim 1, having the load (16), **characterised in that** the operation control circuit (30) is furthermore connected to the load (16) in a controlling manner.

3. Surgical device (10) according to claim 2, **characterised in that** the load (16) is able to be operated in various operating modes in which it has different power consumptions.

4. Surgical device (10) according to claim 2, **characterised in that** the operation control circuit (30) is configured to specify the operating mode of the load (16) and the operation control circuit (30) is furthermore configured to control the power supply unit (21) and the PFC control circuit (29) in accordance with the operating mode of the load (16).

5. Surgical device (10) according to claim 4, **characterised in that** the operation control circuit (30) is configured to set the power supply unit (21) before switching the operating mode to the imminent modified power consumption of the load (16).

6. Surgical device (10) according to one of the preceding claims, **characterised in that** the digital communications interface (31) is formed for electrical isolation.

7. Surgical device (10) according to one of the preceding claims, **characterised in that** the digital communications interface (31) is formed to work bi-directionally.

8. Surgical device (10) according to claim 4 or 5, **characterised in that** the operation control circuit (30) is configured to set the PFC control circuit (29) via the digital communications interface (31) before switching the operating mode to the imminent modified power consumption of the load (16).

9. Surgical device (10) according to one of the preceding claims, **characterised in that** a low-voltage power supply part (26) is connected to the intermediate circuit (20), said low-voltage power supply part being an electrically isolating DC-DC converter.

10. Surgical device (10) according to claim 9, **characterised in that** the electrically isolating DC-DC converter has a forward or flyback converter with a synchronous rectifier (51).

11. Surgical device (10) according to claim 9, **characterised in that** the low-voltage power supply part (26) is controlled by the PFC control circuit (29).

12. Surgical device (10) according to claim 10, **characterised in that** the forward or flyback converter has at least one electronic switch (49) which is connected in series to a primary winding (48) of an electrically isolating transformer (28), wherein the switch (49) is clocked by the PFC control circuit (29).

13. Surgical device (10) according to claim 10, **characterised in that** the synchronous rectifier has an electronic switch (51) having a control electrode which is supplied with switching pulses that are generated by the PFC control circuit (29).

14. Surgical device (10) according to one of the preceding claims, **characterised in that** the PFC control circuit (29) is connected to the DC voltage intermediate circuit (20) via an automatic cut-off starting circuit (57).

15. Method for supplying operating power for a load (16), in particular of an HF generator (15), for supplying a surgical instrument (11),
wherein, with a boost converter (19), which converts mains alternating voltage into an intermediate circuit voltage, an intermediate circuit (20) is supplied, and
wherein, with a power supply part (21), to which electrical energy is supplied from the intermediate circuit (20), the load (16) is supplied,
**characterised in that** a PFC control circuit (29) connected to the boost converter (19) in a controlling manner is supplied with control signals via a digital communications interface (31), said control signals coming from an operation control circuit (30) and identifying load changes, and sends the status signals to the operation control circuit (30).

## Revendications

1. Appareil chirurgical (10) doté d'une charge (16), en particulier appareil chirurgical HF, destiné à l'alimentation d'un instrument chirurgical (11),
comprenant un convertisseur élévateur (19) qui est conçu pour convertir une tension alternative de réseau en une tension de circuit intermédiaire et pour alimenter un circuit intermédiaire à tension continue (20),
comprenant un bloc d'alimentation en puissance (21) qui présente un onduleur (43), connecté au circuit intermédiaire (20), et un transformateur (22) qui présente un enroulement primaire (44), connecté à l'onduleur (43), et un enroulement secondaire (45), en vue de l'alimentation de la charge (16),
comprenant un dispositif de commande (25) qui présente un circuit de commande de fonctionnement (30) connecté au bloc d'alimentation en puissance (30), de manière à le commander,
**caractérisé en ce que** le dispositif de commande (25) présente en outre un circuit de commande PFC (29) connecté au convertisseur élévateur (19), de manière à le commander, et
**en ce que** le circuit de commande PFC (29) et le circuit de commande de fonctionnement (30) sont reliés l'un à l'autre par l'intermédiaire d'une interface de communication (31) numérique.

2. Appareil chirurgical (10) selon la revendication 1, doté de la charge (16), **caractérisé en ce que** le circuit de commande de fonctionnement (30) est en outre connecté à la charge (16), de manière à la commander.

3. Appareil chirurgical (10) selon la revendication 2, **caractérisé en ce que** la charge (16) peut travailler dans différents modes de fonctionnement, dans lesquels elle présente des puissances absorbées différentes.

4. Appareil chirurgical (10) selon la revendication 2, **caractérisé en ce que** le circuit de commande de fonctionnement (30) est conçu pour prédéfinir le mode de fonctionnement de la charge (16), et **en ce que** le circuit de commande de fonctionnement (30) est par ailleurs conçu pour commander l'unité d'alimentation en puissance (21) et le circuit de commande PFC (29), en fonction du mode de fonctionnement de la charge (16).

5. Appareil chirurgical (10) selon la revendication 4, **caractérisé en ce que** le circuit de commande de fonctionnement (30) est conçu pour régler l'unité d'alimentation en puissance (21) sur l'absorption de puissance modifiée imminente de la charge (16), avant la commutation du mode de fonctionnement.

6. Appareil chirurgical (10) selon l'une des revendications précédentes, **caractérisé en ce que** l'interface de communication (31) numérique est réalisée avec isolement de potentiel.

7. Appareil chirurgical (10) selon l'une des revendications précédentes, **caractérisé en ce que** l'interface de communication (31) numérique est réalisée de manière à travailler de façon bidirectionnelle.

8. Appareil chirurgical (10) selon la revendication 4 ou 5, **caractérisé en ce que** le circuit de commande de fonctionnement (30) est conçu pour régler le circuit de commande PFC (29) sur l'absorption de puissance modifiée imminente de la charge (16), par l'intermédiaire de l'interface de communication (31) numérique, avant la commutation du mode de fonctionnement.

9. Appareil chirurgical (10) selon l'une des revendications précédentes, **caractérisé en ce qu'**un bloc d'alimentation très basse tension (26) est connecté au circuit intermédiaire (20), bloc qui est un convertisseur tension continue-tension continue à isolement de potentiel.

10. Appareil chirurgical (10) selon la revendication 9, **caractérisé en ce que** le convertisseur tension continue-tension continue à isolement de potentiel présente un convertisseur à transfert direct ou à transfert indirect, doté d'un démodulateur synchrone (51).

11. Appareil chirurgical (10) selon la revendication 9, **caractérisé en ce que** le bloc d'alimentation très basse tension (26) est commandé par le circuit de commande PFC (29).

12. Appareil chirurgical (10) selon la revendication 10, **caractérisé en ce que** le convertisseur à transfert direct ou à transfert indirect présente au moins un commutateur électronique (49) qui est monté en série avec un enroulement primaire (48) d'un transformateur (28) à isolement de potentiel, le commutateur (49) étant synchronisé par le circuit de commande PFC (29).

13. Appareil chirurgical (10) selon la revendication 10, **caractérisé en ce que** le démodulateur synchrone présente un commutateur électronique (51) doté d'une électrode de commande qui est sollicitée avec des impulsions de communication qui sont produites par le circuit de commande PFC (29).

14. Appareil chirurgical (10) selon l'une des revendications précédentes, **caractérisé en ce que** le circuit de commande PFC (29) est relié au circuit intermédiaire à tension continue (20), par l'intermédiaire d'un circuit de démarrage (57) à coupure automatique.

15. Procédé de mise à disposition d'une puissance de fonctionnement pour une charge (16), en particulier d'un générateur HF (15), en vue de l'alimentation d'un instrument chirurgical (11),
selon lequel un convertisseur élévateur (19), qui convertit une tension alternative de réseau en une tension de circuit intermédiaire, sert à alimenter un circuit intermédiaire (20),
selon lequel un bloc d'alimentation en puissance (21), qui reçoit de l'énergie électrique provenant du circuit intermédiaire (20), sert à alimenter la charge (16),
**caractérisé en ce qu'**un circuit de commande PFC (29), connecté au convertisseur élévateur (19), de manière à le commander, est alimenté, par l'intermédiaire d'une interface de communication (31) numérique, en signaux de commande provenant d'un circuit de commande de fonctionnement (30) et caractérisant des variations de charge, et émet des signaux d'état au circuit de commande de fonctionnement (30).
